# EUROPEAN PATENT SPECIFICATION

(11) **EP 1 409 708 B1**
(45) Date of publication and mention of the grant of the patent: **05.05.2010**
(21) Application number: 02749437.6
(22) Date of filing: 22.07.2002
(51) Int. Cl.: C12P 19/18, C12P 19/08, C12N 15/52, C12N 9/10, C12N 1/20, C12N 1/21, C08B 37/02, A23L 1/054, C12R 1/225

(54) **USES OF A GLUCAN DERIVED FROM LACTIC ACID BACTERIA AS ANTI-CORROSION AGENT**
VERWENDUNG EINES GLUKAN AUS MILCHSÄUREBAKTERIEN ALS KORROSIONSSCHUTZMITTEL
UTILISATION D'UN GLUCANE DERIVE DE BACTERIES D'ACIDE LACTIQUE COMME AGENT ANTICORROSION

(30) Priority: 20.07.2001 EP 01202752; 25.07.2001 EP 01202841
(43) Date of publication of application: 21.04.2004
(73) Proprietor: Nederlandse Organisatie voor Toegepast-Natuurwetenschappelijk Onderzoek TNO, 2628 VK Delft (NL)
(72) Inventor: VAN GEEL-SCHUTTEN, Gerritdina, Hendrika, NL-3971 XG Driebergen (NL)
(74) Representative: van Westenbrugge, Andries
(86) International application number: PCT/NL2002/000495
(87) International publication number: WO 2003/008618

(56) References cited:
- EP-A- 0 427 349
- EP-A- 0 427 349
- WO-A-01/90372
- WO-A-89/12386
- WO-A-96/06173
- US-A- 5 789 209
- US-A- 5 789 209
- VAN GEEL-SCHUTTEN G H ET AL: "Screening and characterization of Lactobacillus strains producing large amounts of exopolysaccharides" APPLIED MICROBIOLOGY AND BIOTECHNOLOGY, SPRINGER VERLAG, BERLIN, DE, vol. 50, no. 6, December 1998 (1998-12), pages 697-703, XP002233876 ISSN: 0175-7598
- UZOCHUKWU SYLVIA ET AL: "Structural analysis by 13C-nuclear magnetic resonance spectroscopy of glucans elaborated by gum-producing bacteria isolated from palm wine" FOOD CHEMISTRY, ELSEVIER SCIENCE PUBLISHERS LTD, GB, vol. 73, no. 2, May 2001 (2001-05), pages 225-233, XP002233877 ISSN: 0308-8146
- PIDOUX M ET AL: "MICROSCOPIC AND CHEMICAL STUDIES OF A GELLING POLYSACCHARIDE FROM LACTOBACILLUS-HILGARDII" CARBOHYDRATE POLYMERS, APPLIED SCIENCE PUBLISHERS, LTD. BARKING, GB, vol. 13, no. 4, 1990, pages 351-362, XP002233878 ISSN: 0144-8617
- MONCHOIS V ET AL: "Cloning and sequencing of a gene coding for a novel dextransucrase from Leuconostoc mesenteroides NRRL B-1299 synthesizing only alpha(1-6) and alpha(1-3) linkages" GENE: AN INTERNATIONAL JOURNAL ON GENES AND GENOMES, ELSEVIER SCIENCE PUBLISHERS, BARKING, GB, vol. 182, no. 1-2, 5 December 1996 (1996-12-05), pages 23-32, XP004071926 ISSN: 0378-1119
- ARGUEELLO-MORALES M A ET AL: "SEQUENCE ANALYSIS OF THE GENE ENCODING ALTERNANSUCRASE, A SUCROSE GLUCOSYLTRANSFERASE FROM LEUCONOSTOC MESENTEROIDES NRRL B-1355" FEMS MICROBIOLOGY LETTERS, AMSTERDAM, NL, vol. 182, 2000, pages 81-85, XP000937860 ISSN: 0378-1097
- GEEL-SCHUTTEN VAN G H ET AL: "EXOPOLYSACCHARIDE PRODUCTION BY LACTOBACILLUS REUTERI INVOLVING SUCRASE TYPE OF ENZYMES" MEDEDELINGEN - FACULTEIT LANDBOUWKUNDIGE EN TOEGEPASTE BIOLOGISCHE WETENSCHAPPEN, UNIVERSITEIT GENT, GENT,, BE, vol. 65, no. 39, 2000, pages 197-201, XP000971140 ISSN: 1373-7503
- MONCHOIS VINCENT ET AL: "Cloning and sequencing of a gene coding for an extracellular dextransucrase (DSRB) from Leuconostoc mesenteroides NRRL B-1299 synthesizing only a alpha(1-6) glucan." FEMS MICROBIOLOGY LETTERS, vol. 159, no. 2, 15 February 1998 (1998-02-15), pages 307-315, XP002198584 ISSN: 0378-1097
- DATABASE EMBL [Online] 2 February 1999 (1999-02-02), "Leuconostoc mesenteroides dextransucrase (DEX) gene, complete cds." XP002360405 retrieved from EBI accession no. EM_PRO:U81374 Database accession no. U81374
- SHIROZA T ET AL: "SEQUENCE ANALYSIS OF THE GTFB GENE FROM STREPTOCOCCUS MUTANS" JOURNAL OF BACTERIOLOGY, WASHINGTON, DC, US, vol. 169, no. 9, September 1987 (1987-09), pages 4263-4270, XP000939115 ISSN: 0021-9193
- DATABASE EMBL [Online] 9 March 1995 (1995-03-09), "Streptococcus salivarius (ATCC 25975) primer-independent glucosyltransferase (gtfL) gene, complete cds." XP002360138 retrieved from EBI accession no. EM_PRO:SSGTFL Database accession no. L35495 -& SIMPSON C L ET AL: "STREPTOCOCCUS SALIVARIUS ATCC 25975 POSSESSES AT LEAST TWO GENES CODING FOR PRIMER-INDEPENDENT GLUCOSYLTRANSFERASES" INFECTION AND IMMUNITY, AMERICAN SOCIETY FOR MICROBIOLOGY. WASHINGTON, US, vol. 63, no. 2, 1 February 1995 (1995-02-01), pages 609-621, XP000647999 ISSN: 0019-9567
- MAASSEN C B M ET AL: "Strain-dependent induction of cytokine profiles in the gut by orally administered Lactobacillus strains" VACCINE, BUTTERWORTH SCIENTIFIC. GUILDFORD, GB, vol. 18, no. 23, May 2000 (2000-05), pages 2613-2623, XP004195926 ISSN: 0264-410X
- DOLS MARGUERITE ET AL: "Characterization of the different dextransucrase activities excreted in glucose, fructose, or sucrose medium by Leuconostoc mesenteroides NRRL B-1299" APPLIED AND ENVIRONMENTAL MICROBIOLOGY, vol. 64, no. 4, April 1998 (1998-04), pages 1298-1302, XP002360116 ISSN: 0099-2240
- VUYST DE L ET AL: "HETEROPOLYSACCHARIDES FROM LACTIC ACID BACTERIA" FEMS MICROBIOLOGY REVIEWS, ELSEVIER, AMSTERDAM, NL, vol. 23, no. 2, 1999, pages 153-177, XP000971896 ISSN: 0168-6445
- PATENT ABSTRACTS OF JAPAN vol. 018, no. 468 (C-1244), 31 August 1994 (1994-08-31) & JP 06 146036 A (NIPPON SYNTHETIC CHEM IND CO LTD:THE;OTHERS: 01), 27 May 1994 (1994-05-27)
- PATENT ABSTRACTS OF JAPAN vol. 2000, no. 02, 29 February 2000 (2000-02-29) & JP 11 310895 A (SUMITOMO METAL IND LTD), 9 November 1999 (1999-11-09)
- KRALJ S ET AL: "Glucan synthesis in the genus Lactobacillus: isolation and characterization of glucansucrase genes, enzymes and glucan products from six different strains" MICROBIOLOGY (READING), vol. 150, no. Part 11, November 2004 (2004-11), pages 3681-3690, XP002360114 ISSN: 1350-0872
- VAN GEEL-SCHUTTEN G H ET AL: "Biochemical and structural characterization of the glucan and fructan exopolysaccharides synthesized by the Lactobacillus reuteri wild-type strain and by mutant strains" APPLIED AND ENVIRONMENTAL MICROBIOLOGY, WASHINGTON,DC, US, vol. 65, no. 7, July 1999 (1999-07), pages 3008-3014, XP002154732 ISSN: 0099-2240
- BINDER T P ET AL: "DISPROPORTIONATION REACTIONS CATALYZED BY LEUCONOSTOC-MESENTEROIDES AND STREPTOCOCCUS-MUTANS GLUCAN SUCRASES" CARBOHYDRATE RESEARCH, vol. 124, no. 2, 1983, pages 275-286, XP002360117 ISSN: 0008-6215
- COTE G L ET AL: "FORMATION OF ALPHA-D-1-3 BRANCH LINKAGES BY A D GLUCAN SUCRASE FROM STREPTOCOCCUS-MUTANS 6715 PRODUCING A SOLUBLE D GLUCAN" CARBOHYDRATE RESEARCH, vol. 127, no. 1, 1984, pages 95-107, XP002360118 ISSN: 0008-6215
- KIM HOSANG ET AL: "Cloning and sequencing of the alpha-1fwdarw6 dextransucrase gene from Leuconostoc mensenteroides B-742CB" JOURNAL OF MICROBIOLOGY AND BIOTECHNOLOGY, vol. 10, no. 4, August 2000 (2000-08), pages 559-563, XP008057614 ISSN: 1017-7825
- KOBAYASHI M ET AL: "STRUCTURAL CHARACTERISTICS OF DEXTRANS SYNTHESIZED BY DEXTRAN SUCRASES EC-2.4.1.5 FROM LEUCONOSTOC-MESENTEROIDES NRRL B-1299" AGRICULTURAL AND BIOLOGICAL CHEMISTRY, vol. 41, no. 10, 1977, pages 1931-1938, XP008057694 ISSN: 0002-1369
- VAN GEEL-SCHUTTEN G H ET AL: "Screening and characterization of Lactobacillus strains producing large amounts of exopolysaccharides." APPLIED MICROBIOLOGY AND BIOTECHNOLOGY, vol. 50, no. 6, December 1998 (1998-12), pages 697-703, XP002233876 ISSN: 0175-7598
- UZOCHUKWU SYLVIA ET AL: "Structural analysis by 13C-nuclear magnetic resonance spectroscopy of glucans elaborated by gum-producing bacteria isolated from palm wine." FOOD CHEMISTRY, vol. 73, no. 2, May 2001 (2001-05), pages 225-233, XP002233877 ISSN: 0308-8146
- PIDOUX M ET AL: "MICROSCOPIC AND CHEMICAL STUDIES OF A GELLING POLYSACCHARIDE FROM LACTOBACILLUS-HILGARDII" CARBOHYDRATE POLYMERS, vol. 13, no. 4, 1990, pages 351-362, XP002233878 ISSN: 0144-8617
- MONCHOIS V ET AL: "Cloning and sequencing of a gene coding for a novel dextransucrase from Leuconostoc mesenteroides NRRL B-1299 synthesizing only alpha(1-6) and alpha(1-3) linkages" GENE: AN INTERNATIONAL JOURNAL ON GENES AND GENOMES, ELSEVIER SCIENCE PUBLISHERS, BARKING, GB, vol. 182, no. 1-2, 5 December 1996 (1996-12-05), pages 23-32, XP004071926 ISSN: 0378-1119
- ARGUEELLO-MORALES M A ET AL: "SEQUENCE ANALYSIS OF THE GENE ENCODING ALTERNANSUCRASE, A SUCROSE GLUCOSYLTRANSFERASE FROM LEUCONOSTOC MESENTEROIDES NRRL B-1355" FEMS MICROBIOLOGY LETTERS, AMSTERDAM, NL, vol. 182, 2000, pages 81-85, XP000937860 ISSN: 0378-1097
- VUYST DE L ET AL: "HETEROPOLYSACCHARIDES FROM LACTIC ACID BACTERIA" FEMS MICROBIOLOGY REVIEWS, ELSEVIER, AMSTERDAM, NL, vol. 23, no. 2, 1999, pages 153-177, XP000971896 ISSN: 0168-6445
- PATENT ABSTRACTS OF JAPAN vol. 018, no. 468 (C-1244), 31 August 1994 (1994-08-31) & JP 06 146036 A (NIPPON SYNTHETIC CHEM IND CO LTD:THE;OTHERS: 01), 27 May 1994 (1994-05-27)
- PATENT ABSTRACTS OF JAPAN vol. 2000, no. 02, 29 February 2000 (2000-02-29) & JP 11 310895 A (SUMITOMO METAL IND LTD), 9 November 1999 (1999-11-09)

## Description

The present invention is in the field of enzymatically produced of biomolecules. The invention is particularly concerned with the use of glucans derived from lactic acid bacteria.

### Background of the invention

Several bacteria are known to produce exopolysaccharides, i.e. polysaccharides secreted into the culture medium. Well-known examples of bacterial exopolysaccharides include xanthan from *Xanthomonas campestris,* gellan from *Sphingomonas paucimobilis* and pullulan from *Aureobasidium pullulans.* Lactic acid bacteria known to produce exopolysaccharides include *Leuconostoc mesenteroides* strains producing dextrans, α(1→6)-linked poly-anhydroglucose, and alternans i.e. poly-anhydroglucoses having alternating α(1→6) and α(1→3)-linkages, oral *Streptococcus* strains producing glucans responsible for dental plaque formation, and a particular *Lactobacillus reuteri* strain producing α(1,6)- and α(1,4)-linked anhydroglucose (Van Geel-Schutten, et al., Appl. Environ. Microbiol. (1999) 65, 3008-3014). The properties of exopolysaccharides depend on the type of monosaccharide units, the type of linkages, the degree and type of branching, the length of the polysaccharide chain, the molecular weight and the conformation of the polymers.

Argiiello-Morales et al. (FEMS Microbiol. Lett. 182 (2000) 81-85) describe an altemansucrase from *Leuconostoc mesenteroides* NRRL B-1355. Monchois et al. (Gene 182 (1996) 23-32; FEMS Microbiol. Lett. 159 (1998) 307-315) for instance describe two different dextransucrases from *Lc. mesenteroides* NRRL B-1299. A method for selecting *Leuconostoc mesenteroides* strains that produce a high proportion of alternan to dextran is described in US 5,789,209. The prior art does not disclose or suggest other lactic acid bacteria than *Leuconostoc* or *Streptococcus* that are capable of producing glucans having both α(1→6) and α(1→3)-linkages.

Several lactic acid bacteria strains were found to be capable of producing a particular class of glucans. These glucans have in common that their anhydroglucose units (AGU) are linked α(1,3)- and/or a(1,6)-glucosidic bonds, i.e. they are α-glucans largely or completely devoid of a(1,4)-bonds. These glucans may be of the alternan (alternating α(1,3) and α(1,6) linkages), mutan (mixed α(1,3) and α(1,6) linkages, usually α(1,3) predominant) or dextran (mainly α(1,6) linkages, some α(1,3) ) type, or other type. The glucans can be produced from sucrose, using sucrase enzymes which are active in the lactic acid bacteria. They can be produced on a large scale and isolated in a commercially feasible way, as the glucans are produced outside the bacterial cell, or even in the absence of the bacteria, using isolated sucrase enzymes. The glucans are produced by food-grade strains and have interesting properties, such as prebiotic utility or thickening of water-based compositions.

The invention is concerned with the use of specific glucans as anticorrosives.

### Description of the invention

*Lactobacillus* strains containing a glucosyltransferase (glucansucrase) capable of producing a glucan having at least 10 anhydroglucose units (AGU) having a backbone consisting essentially of α(1,3)- and/or α(1,6)-linked AGU, in the presence of sucrose can be found among current sources of *Lactobacilli*, such as food sources, silage, mammalian samples etc. These strains containing the glucosyltransferases and producing the glucans can be identified by isolating *Lactobacillus* strains from these sources, growing them on sucrose and analysing the polysaccharide product using suitable analytical methods such as chromatography. The genes encoding these glucosyltransferases can be identified by amplifying nucleotide sequence fragments of the strain using primers based on known glucosyltransferase genes and retaining the positive strains (see examples). Several glucan-producing strains were isolated and identified from different sources and different *Lactobacillus* species, such as *Lb. reuteri, Lb. fermentum, Lb. sake* and *Lb. parabuechneri* or related species. The glucosyltransferases from these glucan-producing strains were also identified and, completely or partly, sequenced (see Examples).

The novel glucans are capable of being produced by glucosyltransferase (glucansucrase) activity of a lactic acid bacterium on a sucrose donor substrate. The glucans have an average molecular weight between 10 kDa and 1 GDa, and consist essentially of α(1,3)- and/or α(1,6)-linked anhydroglucose units (AGU), to which side-chains also consisting of α(1,3)- and/or α(1,6)-linked AGU may be attached.

In particular, the glucans either comprise 15-80% of α(1,3)-linked AGU, 2-80%, especially 4-80% and more especially 15-80% of α(1,6)-linked and 2-25% of α-(1,3,6)-linked (branching) AGU, or 80-99% of α(1,6)-linked AGU and 1-20% of α(1,3)-linked or α-(1,3,6)-linked (branching) AGU, in particular 1-15% of α(1,3)-linked AGU and 5-15% of α(1,3)- and α(1,3,6)-linked units taken together. The invention pertains to the use of a glucan having an average molecular weight of 10 kDa - 50 MDa and comprising 15-26% α(1,3)-linked AGU, 30-50% of α(1,6)-linked AGU, 5-20% of α(1,3,6)-linked AGU and 5-35% of terminal AGU.

Enzymes originating from lactic acid bacteria, or from recombinant sources, capable of producing the glucans described above starting from sucrose can be classified as glucansucrases or glucosyltransferases. More complete sequence information is given in SEQ ID No's 11 and 12.

The enzymes can be used as such for producing the glucans described above, or for producing oligosaccharides and polysaccharides having a similar α(1,3) and/or α(1,6) linked glucan structure. Their genes can also be incorporated in suitable host organisms, to produce alternative glucan-production systems. Such recombinant, preferably food-grade microorganisms, e.g. bacteria, especially lactic acid bacteria, yeasts, fungi etc., containing the genes of the glucansucrases described above are capable of expressing the glucansucrases.

This glucan can be produced by a *Lactobacillus* s train as described above, or by a recombinant micro-organism expressing the glucosyltransferase or by an isolated glucosyltransferase and a suitable glucose source such as for instance sucrose. The glucosyltransferase may be isolated by conventional means from the culture of a glucosyltransferase-positive lactic acid bacterium, especially a *Lactobacillus* species, or from a recombinant organism expressing the glucosyltransferase gene.

The glucan and the gluco-oligosaccharides produced by the *Lactobacillus* strains can be recovered from the culture supernatant of *Lactobacillus* strains described above, containing the glucosyltransferase. The glucan can comprise at least 20, up to about 100,000 α-anhydroglucose units with the unique structure described above.

The glucan-producing enzymes, or at least the most preferred ones, are constitutive in the Lactobacillus strains, in that they are always present. This is contrast to most glucan (dextran-) producing Leuconostoc strains of the prior art, wherein the enzymes are only expressed upon growth in the presence of sucrose. This allows a more efficient production of glucans by the microorganisms.

The glucans of the invention are useful as anticorrosion agents, for example for the protection of ship hulls. For that purpose, they may be applied in the form of solutions or suspensions, by spraying, coating, dipping and other techniques known in the art of corrosion control.

The glucans can be used as such. They can also be modified by physical or chemical means. Suitable examples of chemical modification include oxidation, especially 2,3- or 3,4-oxidation using periodate or hypohalite, in glucans having α-1,6 linkages, or 6-oxidation using nitroxyls with peracid or hypohalite in glucans having α-1,3 linkages. Hypohalite oxidation resulting in ring-opened 2,3- or 3,4-dicarboxy-anhydroglucose units (see e.g. EP-A-427349), while periodate oxidation results in ring-opened 2,3- or 3,4-dialdehyde-anhydroglucose units (see e.g. WO 95/12619), which can be further oxidised to (partially) carboxylated units (see e.g. WO 00/26257). Nitroxyl-mediated oxidation using hypochlorite or a peracid results in 6-aldehyde- and 6-carboxy-anhydroglucose units (see e.g. WO 95/07303).

The oxidised glucans have improved water-solubility, altered viscosity and a retarded fermentability and can be used as metal-complexing agents, detergent additives, strengthening additives, bioactive carbohydrates, emulsifiers and water binding agents. They can also be used as starting materials for further derivatisation such as cross-linking and the introduction of hydrophobes. Oxidised glucans coupled to proteins can be used as emulsifiers and stabilisers. The oxidised glucans preferably contain 0.05-1.0 carboxyl groups, more preferably 0.2-0.8 carboxyl groups per anhydroglucose unit, e.g. as 6-carboxyl groups on 1 ,3-linked units.

When modified glucans with high proportion of carboxyl groups are desired, two oxidation processes can be combined or an oxidation can be combined with e.g. carboxymethylation (see below). Thus, an α-(1,3/1,6)-glucan having a degree of substitution (DS) for carboxyl groups between 0,3 and 1,0 can be conveniently prepared by first nitroxyl-mediated oxidation, resulting in 1,3-substituted units being oxidation to glucuronic acid units, followed by e.g. periodate and chlorite oxidation, resulting in 1,6-substituted units* being converted to ring-opened dicarboxy-substituted units. The order of processes can also be inverted, or one oxidation process, such as nitroxyl-mediated 6-oxidation can be combined with carboxymethylation. Also, by appropriate adaptation of the oxidation processes mixed aldehyde-containing and carboxyl-containing polymers can be obtained.

Other useful modifications are alkylation, acylation, hydroxyalkylation, amino-alkylation, carboxyalkylation, phosphorylation, sulphatation, as well as physical and chemical crosslinking. Phosphorylation (see: O.B. Wurzburg (1986), Modified Starches: properties and uses. CRC Press Inc., Boca Raton, 97-112) can be achieved by dry heating glucans with a mixture of monosodium and disodium hydrogen phosphate or with tripolyphosphate. The phosphorylated glucans are suitable as wet-end additives in papermaking, as binders in paper coating compositions, as warp sizing-agents, and as core binders for sand molds for metal casting. Acylation, especially acetylation or propionylation using acetic or propionic anhydride respectively, results in products suitable as bleaching assistants and for the use in foils. Acylation with e.g. alkenyl succinic anhydrides or (activated) fatty acids results in surface-active products suitable as e.g. surfactants, emulsifiers, and stabilisers. Crosslinking, e.g. by coupling oxidised derivatives, or by reaction with a crosslinking agent such as triphosphoric acid, epichlorohydrine or a dialdehyde, can be used to adjust the physical properties of the glucans, e.g. to enhance their water-binding or thickening capacities.

Hydroxyalkylation is commonly performed by base-catalysed reaction with alkylene oxides, such as ethylene oxide, propylene oxide or epichlorohydrin; the hydroxyalkylated products have improved solubility and viscosity characteristics. Carboxymethylation is achieved by reaction of the glucans with monochloroacetic acid or its alkali metal salts and results in anionic polymers suitable for various purposes including crystallisation inhibitors, and metal complexants. Amino-alkylation can be achieved by reaction of the glucans with alkylene-imines, halo-alkyl amines or amino-alkylene oxides, or by reaction of epichlorohydrine adducts of the glucans with suitable amines. These products can be used as cationic polymers in a variety of applications, especially as a wet-end additive in paper making to increase strength, for filler and fines retention, and to improve the drainage rate of paper pulp. Other potential applications include textile sizing and wastewater purification. The above mentioned modifications can be used either separately or in combination depending on the desired product. Furthermore, the degree of chemical modification is variable and depends on the intended use. If necessary 100% modification, i.e. modification of all anhydroglucose units can be performed. However, partial modification, e.g. from less than 1 (e.g. 0.2) modified anhydroglucose unit per 100 units up to higher levels, will often be sufficient in order to obtain the desired effect.

Another suitable type of derivatives is formed by hydrolysates of the present glucans. Hydrolysis can be performed in a controlled manner in a way known per se, using e.g. dilute acid or glucanolytic enzymes, especially α-1,3-glucanases or α-1,6 glucanases. Hydrolysis results in polysaccharides of reduced chain length (degree of polymerisation, DP, of more than 20) or oligosaccharides (DP of less than 20).

Gluco-oligosaccharides containing the characteristic structure of the glucan described above can be produced using an isolated glucansucrase or a *Lactobacillus* strain, or a recombinant micro-organism containing (a part of) a glucosyltransferase. The production of the gluco-oligosaccharides is different from the glucan synthesis reaction. In addition to sucrose, the substrate of the glucansucrase, an acceptor molecule such as maltose or lactose can be used as an acceptor, to synthesise oligosaccharides. Consecutive attachment of glucose units in a manner determined by the particular glucansucrase results in α(1,3)- and/or α(1,6)-linked gluco-oligosaccharides, the chain length of which can be determined by selecting the appropriate reaction conditions. Longer reaction times, higher sucrose levels and lower acceptor levels will usually result in relatively long chains, e.g. having a degree of polymerisation (DP) of more than 10, up to several hundreds if desired, while shorter reaction times, lower sucrose levels and higher acceptor levels will result in relatively short chains, e.g. with a DP from about 3 up to 10 or higher. Another way of producing gluco-oligosaccharides is by hydrolysis of the glucan described above. This hydrolysis can be performed by known hydrolysis methods such as enzymatic hydrolysis with enzymes such as amylase, dextranase or pullulanase or by acid hydrolysis. The produced gluco-oligosaccharides contain at least one 1,6- or one 1,3-glucosidic link to be used as prebiotics.

### Examples

### General

The various lactic acid bacterial strains were isolated form a variety of sources, including fermented foods, the gastrointestinal tract of various human or animal species, and silage.

### Example 1: Identification and nucleotide sequence of glucansucrase/glucosyltransferase genes from lactobacilli

The glucansucrase genes were identified by amplification with PCR using degenerated primers (GTFrev, 5' ADRTC NCCRT ARTAN AVNYK NG 3' and GTFforw, 5'-GAYAAYWSNA AYCCNRYNGT NC-3'; N = A, C, G or T, Y = T or C, K = G or T, W = A or T, S = C or G, R = A or G), based on conserved amino acid sequences of different published glucansucrase genes. An amplification product with the predicted size of about 660 bp was obtained and cloned in *Escherichia coli* Top 10 using pCR-XL-TOPO (Invitrogen). Sequence analysis confirmed that part of a *gtf* gene had been isolated. The 660 bp amplified was used to design primers for inversed PCR. For inverse PCR chromosomal DNA was digested with 10 different enzymes ligated, yielding circular DNA molecules. PCR with the diverging primers with the circular ligation products as template yielded amplicons of various sizes, those products were cloned into pCR-XL-TOPO (Invitrogen) and sequenced (GATC, Konstanz, Germany). If necessary additional inverse PCR reactions were carried out to obtain the complete gene(s). Both strands of the entire glucansucrase genes were sequenced twice.

### Example 2: Isolation and identification of α-(1,6) glucan and a glucansucrase from Lactobacillus reuteri strain 180

L. reuteri strain 180 was deposited as LMG P-18389 at the BCCM/LMG Culture Collection at Gent, Belgium. The strain was grown in 18 litres of MRS-s medium (in g per kg): yeast extract (22), sodium acetate trihydrate (5), sodium citrate dihydrate (2.42), ammonum chloride (1.32), dipotassium hydrogen phosphate (2), magnesium sulphate heptahydrate (0.2), manganese sulphate heptahydrate (0.05), sorbitan mono-oleate (1), vitamins (in mg per kg: B1: 14.4, B2: 3.6, B3: 72, H 0.216), sucrose (100), tap water (remainder), for 21 h at 37°C under anaerobic conditions (pH 5.5). See also: Van Geel-Schutten et al., Appl. Microbiol. Biotechnol. (1998) 50, 697-703. During growth, 13 g/l polysaccharide was produced. This polysaccharide was isolated as described in the reference cited above. The monosaccharide composition of the polysaccharide was determined by hydrolysis of the soluble part of the polysaccharide and high-performance anion-exchange chromatography. It was characterised as a glucan. This glucan was not formed when the strain was grown on glucose instead of sucrose. Methylation analysis (Van Geel-Schutten et al. 1999) revealed the presence of 17-24% α(1,3)-linked glucosyl units, 34-44% of α(1,6)-linked glucosyl units, 7-15% of α(1,3,6)-linked glucosyl units and 7-35% of terminal glucosyl units. The average molecular weight of the glucan was determined to be 3.6 x 10⁷ Da and the Rg was 45 nm.

The average molecular weight of the polysaccharide was established using the SEC-MALLS system: 0.0522 g of the glucan was dissolved in 10 ml DMSO/water (90/10) and heated for 1 hour at 80°C, filtered through a 0.45µm filter and injected on the SEC-MALLS system and analysed using the following conditions:

| | |
|---|---|
| Eluent: | DMSO/water (90/10) with 0.1 M NaNO₃ |
| Flow rate: | 0.5 ml/min |
| Injection volume: | 0.247 ml |
| Column: | PLgel Guard, mixed-A and mixed-D |
| Temperature: | 90°C |

Detection: MALLS (DAWN-DSP), 50°C, A₂=0, dn/dc=0.074, F2 cell, RI; SDS PAGE followed by PAS-staining (Van Geel-Schutten et al. 1999) revealed the presence of an extracellular sucrase with a molecular weight of about 190 kDa. Part of the gene encoding the sucrase enzyme was isolated using PCR techniques and sequenced. On the deduced amino acid sequence of the fragment, high homologies were found with other glucansucrases. This partial sequence information is given in SEQ ID No. 1 (DNA) and 2 (protein). Full sequence information is given in SEQ ID No's. 11 and 12.

The glucan produced by *L. reuteri* strain 180 has been tested for application on ship hulls for the prevention of corrosion (see Example 4).

### Example 3: Isolation and identification of α-(1,6/1,3) glucan and a glucansucrase from Lactobacillus reuteri strain ML1

*L. reuteri* strain ML1, deposited as LMG P-20347 at the BCCM/LMG Culture Collection at Gent, Belgium, was grown overnight under anaerobic conditions at 37°C on MRS supplemented with sucrose (see Example 2). The cells were removed by centrifugation and two volumes of ethanol were added to the supernatant. The precipitated polysaccharides were harvested by centrifugation and resuspended in 2-3 liters of demi water and precipitated again with two volumes of ethanol. The glucan produced by this strain (7 g) was characterised by methylation analysis and monosaccharide composition analysis as described in Example 2. The polymer was found to consist of 48-53% of α(1-3) linked glucosyl units, 3-8% of α(1-6) linked glucosyl units, 12-20% of α(1-3-6) linked glucosyl units (branching units) and 20-30% of 1-linked (terminal) glucose units. The glucans were not produced during growth on glucose. The average molecular weight of the polysaccharide was established to be 7.6x10⁶ Da using the SEC-MALLS system as described in example 2. These were the first examples of the production ofmutan-like polymers by lactobacilli. The glucan produced by *L. reuteri* strain ML1 has been tested for application as anticorrosive agent and showed excellent utility for the prevention of corrosion e.g. on ship hulls.

SDS PAGE followed by PAS-staining (Van Geel-Schutten et al. 1999) revealed the presence of an extracellular sucrase with a molecular weight of about 190 kDa. It was found that this strain produces two glucansucrases.

### Example 4: Anticorrosion properties of glucans

Plain carbon steel sheets of 1 cm² embedded in an epoxy matrix were exposed to a slightly corrosive medium (150 ml of 0.1 M LiClO₄) with or without the addition of a bacterial polysaccharide (0.2 g) for several days. The sheets were then examined visually and electrochemically from time to time. The corrosion potential (E_{corr} in mV with reference to Ag/AgCl) and polarisation resistance (Rₚ in kΩ/cm²) are both a measure of the anti-corrosion effect. After an initial adaptation of 3-10 hours, these parameters attained a stable value. The experiments were carried with a heteropolysaccharide from *Lactobacillus sake*, and a homopolysaccharide of the invention (from LB 180 according to example 2), as well as without polysaccharide. The results are summarised in the table below. It follows that the anti-corrosion properties of the glucan of the invention are superior. It was found that the homopolysaccharide of ML 1 (example 3) has at least equal anticorrosion performance as the LB 180 polysaccharide.

**Table: Corrosion experiments**

| organism | type of polysaccharide | aspect of treated sheet | E_{corr} (mV vs. Ag/AgCl) | Rₚ (kΩ/cm²) |
|---|---|---|---|---|
| control | - | corrosion | -700 | 1.5 |
| *Lb. sake* | hetero-polysaccharide | localised corrosion | -600 | 4.5 |
| *Lb. 180* | α-glucan | thin black layer | -200 | 70 |

### Example 5: Modification of α-1, 3/1,6-glucan by oxidation

One gram (6.15 mmol of anhydroglucose units) of the α-1,3/1,6-glucan produced by strain LB 33 is resuspended in 100 ml water. Next, 2,2,6,6-tetramethylpiperidine-1-oxyl (TEMPO; 0.01 g, 0.065 mmol) and sodium bromide (100 mg, 1 mmol) are added and the suspension is cooled to 0°C. The reaction can also be performed without bromide. A solution of hypochlorite (3 ml, 15% solution, 6.3 mmol) of pH 10.0 (0°C) is added. The pH is kept constant by addition of 0.1M NaOH. After 1 hr, the solution is poured into 150 ml 96% ethanol, causing the product to precipitate. The white precipitate is centrifuged, resuspended in ethanol/water (70/30 v/v) and centrifuged again. Next, the precipitate is resuspended in 96% ethanol, centrifuged and dried. The uronic acid content is determined by means of the uronic acid assay according to Blumenkrantz and Abdoe-Hansen (Anal. Biochem. 54 (1973), 484). A calibration curve was generated using polygalacturonic acid (5, 10, 15 and 20 µg). With this calibration curve the uronic acid content in a sample of 20 µg of the product is determined. The major part of 6-hydroxyl groups have been oxidised to carboxyl groups.

### Example 6: Construction of plasmids for expression of the glucansucrase genes in E. coli.

Two primers were designed with appropriate restriction sites; the C-terminal primer contained in all cases a His-tag. The PCR products were first cloned in pCR-XL-TOPO. The PCR products were removed from pCR-XL-TOPO using the appropriate enzymes and ligated in the appropriate sites of an expression vector (e.g pET15b (Novagen)). For the expression of part of the glucosyltransferase gene of LB 180 (for better expression, the N-terminal region encoding the N-terminal variable domain of the glucansucrase,was not cloned) in *E. coli*, a PCR reaction was performed using Forw180 ( 5'-GATGCAT**GAG CTC**CCATGGG CATTAACGGC CAACAATATT ATTATTGACC C-3') containing *Sac*I (bold) and *Nco*I (underlined) sites, and Rev180 (5'-ATATCGAT**GG GCCC**CGGATC CTATTAGT*GA TGGTGATGGT GATGTT*TTTG GCCGTTTAAA TCACCAGGTT TTAATGG-3'), containing *Apa*I (bold), *Bam*HI (underlined) and a 6x His-tag (italics) as primers. The PCR product was cloned in pCR-XL-TOPO. The PCR product was removed from pCR-XL-TOPO using *Nco*I/*Bam*HI and ligated in the coresponding sites of pET15b (Novagen). The resulting plasmid (pET15b180) containing part of the glucansucrase gene of 704 amino acids encoding a glucansucrase without the variable N-terminal domain was transformed to *E. coli* B121 DE3 star (Invitrogen).

Cells of *E. coli* harbouring the pET15b180 were harvested by centrifugation after 16 h of growth under aerobic conditions at 37 °C. The pellet was washed with 50 mM sodium acetate buffer pH 5.5 containing 1 mM CaCl₂ and 1% (v/v) Tween 80 and the suspension was centrifuged again. Pelleted cells were resuspended in with 50 mM sodium acetate buffer pH 5.5 containing 1 mM CaCl₂ and 1% (v/v) Tween 80, and 7.2 mM β-mercaptoethanol. Cells were broken by sonication and cell debris and intact cells were removed by centrifugation for 15 minutes at 4 °C at 14,000 rpm (Eppendorf). The resulting cell free extract was used as enzyme source to produce high molecular weight glucans from sucrose in 50 mM sodium acetate buffer pH 5.5 containing 1 mM CaCl₂ and 1% (v/v) Tween 80 and 10 g/l sucrose. After 16 hours of incubation, the glucans were isolated using ethanol precipitation. When cell free extracts of *E. coli* B121 DE3 star (Invitrogen) harbouring the plasmid pET15b (without insert) were used as enzyme source, no glucans were produced from sucrose.

### Sequence information

SEQ ID No's 1 and 2 give the nucleotide and amino acid sequence, respectively, of a part of the glucansucrase from strain Lb180 as originally determined (Example 2). The partial sequence shows 53% (199/223) sequence identity and 68% similarity with dextransucrase DSRB742 of *Leuconostoc mesenteroides (Lc. mes.),* with 2 gaps (between amino acids F172 and N173), and 52% identity with some other dextransucrases and alternansucrases *of Lc. mes*.

SEQ ID No's 11 and 12 give the nucleotide and amino acid sequence, respectively, of the glucansucrase of strain Lb180 (Example 2). The sequence shows 1322/1768 (74%) sequence identity and 1476/1768 (82%) similarity with 15/1768 gaps with glucansucrase from *Lb. reuteri* LB 121 as disclosed in WO 01/90372. The -35 and -10 sites TTGAAA and TATAA are located at nucleotide positions 561 and 599, respectively. The ribosome binding site (RBS) GAAGGAG is at 574 and the start codon ATG at 587. Inverted repeats AAGCAGCTC and GAGCTGCTT are at 6025 and 6051. Possible stop codons (TAA, TAG, TGA) are indicated with an * (5963).

### SEQUENCE LISTING

<110> TNO
<120> Novel glucans and novel glucansucrases derived from lactic acid bacteria
<130> Novel glucans and glucansucrases
<140>
   <141>
<160> 10
<170> PatentIn Ver. 2.1
<210> 1
   <211> 665
   <212> DNA
   <213> Lactobacillus reuteri
<400> 1
   gataatacga atccggtggt gcaagctgaa gaattaaact ggctttacta tttaatgaat 60
   ttcggtacaa ttacaggaaa taatcctgaa gctaattttg atggtattcg agtggatgct 120
   gttgataatg tagatgttga cttattgagt attgcacgtg attactttaa tgcagcatat 180
   aacatggagc aaagtgatgc cagtgctaat aagcacatta atattttgga agattgggga 240
   tgggatgatc ctgcttatgt aaataagatt ggaaatcctc aattaacaat ggatgatcgt 300
   ttacgaaatg caattatgga tacattatca ggagcacctg ataaaaacca agcattgaat 360
   aaattaatta ctcagtcatt agtaaatcgt gctaatgata atactgaaaa cgcggttatt 420
   ccaagctata attttgttcg agcacatgat agtaatgctc aagaccaaat tcgtcaggct 480
   attcaagctg caactggaaa accatatggc gaatttaact tagatgatga aaagaagggt 540
   atggaagcat atattaatga tcagaattct actaataaga agtggaatct ttacaatatg 600
   ccttctgctt atactattct tctaacaaat aaagattcag ttccccacgt ctactatggc 660
   gacat                                                             665
<210> 2
   <211> 221
   <212> PRT
   <213> Lactobacillus reuteri
<400> 2
   Asp Asn Thr Asn Pro Val Val Gln Ala Glu Glu Leu Asn Trp Leu Tyr
    1               5                  10                  15
   Tyr Leu Met Asn Phe Gly Thr Ile Thr Gly Asn Asn Pro Glu Ala Asn
               20                  25                  30
   Phe Asp Gly Ile Arg Val Asp Ala Val Asp Asn Val Asp Val Asp Leu
           35                  40                  45
   Leu Ser Ile Ala Arg Asp Tyr Phe Asn Ala Ala Tyr Asn Met Glu Gln
       50                  55                  60
   Ser Asp Ala Ser Ala Asn Lys His Ile Asn Ile Leu Glu Asp Trp Gly
    65                  70                  75                  80
   Trp Asp Asp Pro Ala Tyr Val Asn Lys Ile Gly Asn Pro Gln Leu Thr
                   85                  90                  95
   Met Asp Asp Arg Leu Arg Asn Ala Ile Met Asp Thr Leu Ser Gly Ala
                   100                 105                 110
   Pro Asp Lys Asn Gln Ala Leu Asn Lys Leu Ile Thr Gln Ser Leu Val
           115                 120                 125
   Asn Arg Ala Asn Asp Asn Thr Glu Asn Ala Val Ile Pro Ser Tyr Asn
       130                 135                 140
   Phe Val Arg Ala His Asp Ser Asn Ala Gln Asp Gln Ile Arg Gln Ala
   145                 150                 155                 160
   Ile Gln Ala Ala Thr Gly Lys Pro Tyr Gly Glu Phe Asn Leu Asp Asp
                   165                 170                 175
   Glu Lys Lys Gly Met Glu Ala Tyr Ile Asn Asp Gln Asn Ser Thr Asn
               180                 185                 190
   Lys Lys Trp Asn Leu Tyr Asn Met Pro Ser Ala Tyr Thr Ile Leu Leu
           195                 200                 205
   Thr Asn Lys Asp Ser Val Pro His Val Tyr Tyr Gly Asp
       210                 215                 220
<210> 3
   <211> 674
   <212> DNA
   <213> Lactobacillus strain LB 33
<400> 3
   gacaattcga atccggtggt gcaagcggaa cagttaaact ggttatacta catgatgaat 60
   ataggtagca ttactgccaa tgatcccacc gcaaactttg atggctatcg agtggacgct 120
   gtggacaatg tcgatgctga tttattaaat atagctgccg attatgccaa agatgcttat 180
   aaaactaatc aaagtgatgc taatgccaac aaacatttat caatattaga agattgggat 240
   aataatgatc cggcttatat caaagcacat ggaaataatc agttaactat ggatttccca 300
   gcacatttag caattaaata ttcattaaat atgccagtaa gtcaacgaag tgggctggaa 360
   ccagagctca caaccagttt agttaacaga actggtgatg attctactga aaatgtcgca 420
   cagccaaact atacttttat tagggctcac gatagtgaag tgcaaacaat catcgcacaa 480
   attatcaaag ataaaatcaa ccctaactct gacggattaa cagttactcc cgatgaaata 540
   agtcaggcct ttaaaatata taatgcagat gaattaaaga ctgataaaca atatactttt 600
   tataacatgc cctctgccta tactattttg ctaaccaata aagatacagt accccacctc 660
   tattacggcg acat                                                   674
<210> 4
   <211> 224
   <212> PRT
   <213> Lactobacillus strain LB 33
<400> 4
   Asp Asn Ser Asn Pro Val Val Gln Ala Glu Gln Leu Asn Trp Leu Tyr
   1 5 10 15
   Tyr Met Met Asn Ile Gly Ser Ile Thr Ala Asn Asp Pro Thr Ala Asn
   20 25 30
   Phe Asp Gly Tyr Arg Val Asp Ala Val Asp Asn Val Asp Ala Asp Leu
   35 40 45
   Leu Asn Ile Ala Ala Asp Tyr Ala Lys Asp Ala Tyr Lys Thr Asn Gln
   50 55 60
   Ser Asp Ala Asn Ala Asn Lys His Leu Ser Ile Leu Glu Asp Trp Asp
   65 70 75 80
   Asn Asn Asp Pro Ala Tyr Ile Lys Ala His Gly Asn Asn Gln Leu Thr
   85 90 95
   Met Asp Phe Pro Ala His Leu Ala Ile Lys Tyr Ser Leu Asn Met Pro
   100 105 110
   Val Ser Gln Arg Ser Gly Leu Glu Pro Glu Leu Thr Thr Ser Leu Val
   115 120 125
   Asn Arg Thr Gly Asp Asp Ser Thr Glu Asn Val Ala Gln Pro Asn Tyr
   130 135 140
   Thr Phe Ile Arg Ala His Asp Ser Glu Val Gln Thr Ile Ile Ala Gln
   145 150 155 160
   Ile Ile Lys Asp Lys Ile Asn Pro Asn Ser Asp Gly Leu Thr Val Thr
   165 170 175
   Pro Asp Glu Ile Ser Gln Ala Phe Lys Ile Tyr Asn Ala Asp Glu Leu
   180 185 190
   Lys Thr Asp Lys Gln Tyr Thr Phe Tyr Asn Met Pro Ser Ala Tyr Thr
   195 200 205
   Ile Leu Leu Thr Asn Lys Asp Thr Val Pro His Leu Tyr Tyr Gly Asp
   210 215 220
<210> 5
   <211> 671
   <212> DNA
   <213> Leuconostoc strain 86
<400> 5
   gacaacacga acccggtggt gcaagctgag cagttaaatt ggttacacta tttgatgaat 60
   tttggtagca ttacagcaaa cgattctgct gctaattttg atgagatacg tgtcgatgct 120
   gtcgataatg ttgacgctga tttactccag attgcagcag attatttcaa agctgcttat 180
   ggtgttgata aaaatgacgc aacagcaaat caacatcttt caattcttga agattggagc 240
   cataacgacc ctgaatacgt gaaggatttt ggtaataatc aactcacaat ggatgattac 300
   atgcataccc agttaatctg gtcgttgact aaagatatgc gtatgcgtgg taccatgcaa 360
   cgcttcatgg actattacct cgtcaatcgc aatcacgata gtaccgaaaa cactgccatt 420
   ccaaattaca gctttgttcg cgcacacgat agtgaagtac aaacagtcat tgctcaaatt 480
   atttctgagt tacatcccga cgtaaaaaat agtttggcac caacagcaga ccagctagcc 540
   gaagccttta aagtttataa taacgatgaa aaacaggcgg ataagaaata tacacaatac 600
   aacatgccta gcgcctatgc gatgctgtta actaataaag atacagtacc ccgcgtctac 660
   tatggcgaca t                                                       671
<210> 6
   <211> 223
   <212> PRT
   <213> Leuconostoc strain 86
<400> 6
   Asp Asn Thr Asn Pro Val Val Gln Ala Glu Gln Leu Asn Trp Leu His
   1               5                   10                  15
   Tyr Leu Met Asn Phe Gly Ser Ile Thr Ala Asn Asp Ser Ala Ala Asn
               20                  25                  30
   Phe Asp Glu Ile Arg Val Asp Ala Val Asp Asn Val Asp Ala Asp Leu
           35                  40                  45
   Leu Gln Ile Ala Ala Asp Tyr Phe Lys Ala Ala Tyr Gly Val Asp Lys
       50                  55                  60
   Asn Asp Ala Thr Ala Asn Gln His Leu Ser Ile Leu Glu Asp Trp Ser
   65                  70                  75                  80
   His Asn Asp Pro Glu Tyr Val Lys Asp Phe Gly Asn Asn Gln Leu Thr
                   85                  90                  95
   Met Asp Asp Tyr Met His Thr Gln Leu Ile Trp Ser Leu Thr Lys Asp
               100                 105                 110
   Met Arg Met Arg Gly Thr Met Gln Arg Phe Met Asp Tyr Tyr Leu Val
           115                 120                 125
   Asn Arg Asn His Asp Ser Thr Glu Asn Thr Ala Ile Pro Asn Tyr Ser
       130                 135                 140
   Phe Val Arg Ala His Asp Ser Glu Val Gln Thr Val Ile Ala Gln Ile
   145                 150                 155                 160
   Ile Ser Glu Leu His Pro Asp Val Lys Asn Ser Leu Ala Pro Thr Ala
                   165                 170                 175
   Asp Gln Leu Ala Glu Ala Phe Lys Val Tyr Asn Asn Asp Glu Lys Gln
               180                 185                 190
   Ala Asp Lys Lys Tyr Thr Gln Tyr Asn Met Pro Ser Ala Tyr Ala Met
           195                 200                 205
   Leu Leu Thr Asn Lys Asp Thr Val Pro Arg Val Tyr Tyr Gly Asp
       210                 215                 220
<210> 7
   <211> 746
   <212> DNA
   <213> Leuconostoc strain 86
<400> 7
   gactcactat agggcgaatt gggccctcta gatgcatgct cgagcggccg ccagtgtgat 60
   ggatatctgc agaattcgcc cttgacaatt ggaatccggt ggtgcaagct gaacagttga 120
   attggctaca ctatttgatg aatcttggaa cgattacagc taatgatcca gatgctaatt 180
   ttgacagcat aagagttgac gctgtcgata atgtggatgc agatttgtta gatattgcac 240
   gtgattactt taatgcagta tacaaggtta accaaagtga tgttaatgct aataaacata 300
   tttctatttt agaagattgg agtggattag atcccaatga ggttgttaaa aatgggaatc 360
   cacaattaac acttaacaca ggggttcaaa attcattatt aaatgctttg acaaaggggc 420
   caaataatcg ttggggggat agactcattg attgataaat caacaatgag atatccagat 480
   aaggatggta aaatccttat tcctaattat agtttcgtac gtgcacacga tagtgaagtt 540
   caaggtatta ttggcaaata ttaacagatc atacgtcagc cgaatcaggt aataaattca 600
   caaaggatcc attaaaacag gcttggatta ttactatgct gaatcaagaw tagactgtta 660
   aagaatattc gcactataat atggcgagtg cttatgcagc attgttaaca aattaagata 720
   ccattcccaa ctctactacg gcgact                                      746
<210> 8
   <211> 221
   <212> PRT
   <213> Leuconostoc strain 86
<400> 8
   Asp Asn Trp Asn Pro Val Val Gln Ala Glu Gln Leu Asn Trp Leu His
   1               5                   10                  15
   Tyr Leu Met Asn Leu Gly Thr Ile Thr Ala Asn Asp Pro Asp Ala Asn
               20                  25                  30
   Phe Asp Ser Ile Arg Val Asp Ala Val Asp Asn Val Asp Ala Asp Leu
           35                  40                  45
   Leu Asp Ile Ala Arg Asp Tyr Phe Asn Ala Val Tyr Lys Val Asn Gln
       50                  55                  60
   Ser Asp Val Asn Ala Asn Lys His Ile Ser Ile Leu Glu Asp Trp Ser
   65                  70                  75                  80
   Gly Leu Asp Pro Asn Glu Val Val Lys Asn Gly Asn Pro Gln Leu Thr
                   85                  90                  95
   Leu Asn Thr Gly Val Gln Asn Ser Leu Leu Asn Ala Leu Thr Lys Gly
               100                 105                 110
   Pro Asn Asn Arg Trp Gly Ile Asp Ser Leu Ile Asp Lys Ser Thr Met
           115                 120                 125
   Arg Tyr Pro Asp Lys Asp Gly Lys Ile Leu Ile Pro Asn Tyr Ser Phe
       130                 135                 140
   Val Arg Ala His Asp Ser Glu Val Gln Gly Ile Ile Gly Lys Ile Leu
   145                 150                 155                 160
   Thr Asp His Thr Ser Ala Glu Ser Gly Asn Lys Phe Thr Lys Asp Gln
                   165                 170                 175
   Leu Lys Gln Ala Leu Asp Tyr Tyr Tyr Ala Asp Gln Asp Lys Thr Val
               180                 185                 190
   Lys Glu Tyr Ser His Tyr Asn Met Ala Ser Ala Tyr Ala Ala Leu Leu
           195                 200                 205
   Thr Asn Lys Asn Thr Ile Pro Asn Leu Tyr Tyr Gly Asp
       210                 215                 220
<210> 9
   <211> 670
   <212> DNA
   <213> Leuconostoc strain 86
<400> 9
   gataattcga atccgatggt gcaagctgag caattgaact ggcttcacta catgatgaac 60
   attggtacta tagctcagaa cgacccaaca gctaattttg acggttatcg tgttgatgcg 120
   gttgataatg ttgatgccga tctcttacaa attgctggtg attactttaa agctgcatac 180
   ggtactggta aaactgaggc aaacgcaaac aatcatattt cgatcttgga agattgggat 240
   aataatgatt ctgcgtacat taaagcccac gggaacaacc aattgacaat ggattttcca 300
   gcacacttgg ctttgaaata cgccttgaac atgcctcttg ccgcacaaag tggcctagaa 360
   ccgctaatta atacaagtct tgttaagcgt gggaaagatg ccacagaaaa tgaagcacaa 420
   ccaaactatg cctttatccg tgcccatgat agtgaagtgc agacagttat tgcacaaatt 480
   attaaggata aaattaacac aaaatcagac ggcttaactg taacaccaga tgagattaag 540
   caagctttca atatttacaa cgccgatgaa ttaaaagcag ataaggaata tacagcatac 600
   aatattcctg cttcttacgc tgtattgttg acaaacaagg atactgtgcc cgcgtctact 660
   atggcgacct 670
<210> 10
   <211> 223
   <212> PRT
   <213> Leuconostoc strain 86
<400> 10 SEQ ID No. 11 DNA
   SEQ ID No. 12 PRT
   Lactobacillus reuteri strain 180 SEQ ID No. 13 DNA
   SEQ ID No. 14 PRT
   Lactobacillus reuteri strain ML1 SEQ ID No. 15 DNA
   SEQ ID No. 16 PRT
   Lactobacillus reuteri strain ML1 (ML4) SEQ ID No. 17 DNA
   Lactobacillus strain LB33 SEQ ID No. 18 PRT
   Lactobacillus strain LB33 SEQ ID No. 19 DNA
   SEQ ID No. 20 PRT
   Lactobacillus sake strain KG15 SEQ ID No. 21 DNA
   SEQ ID No. 22 PRT
   Lactobacillus fermentum strain LB33

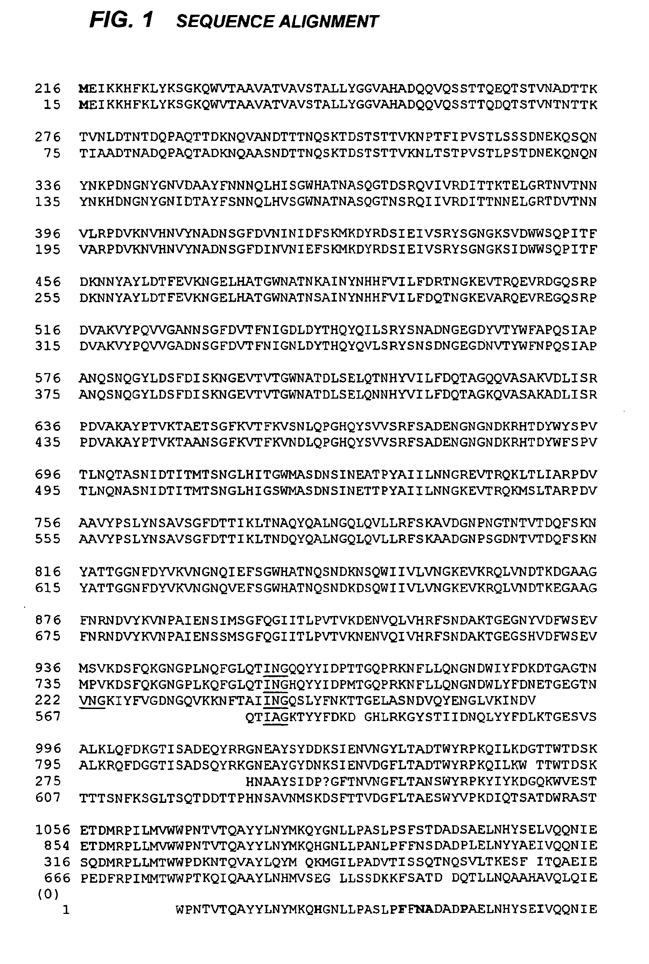

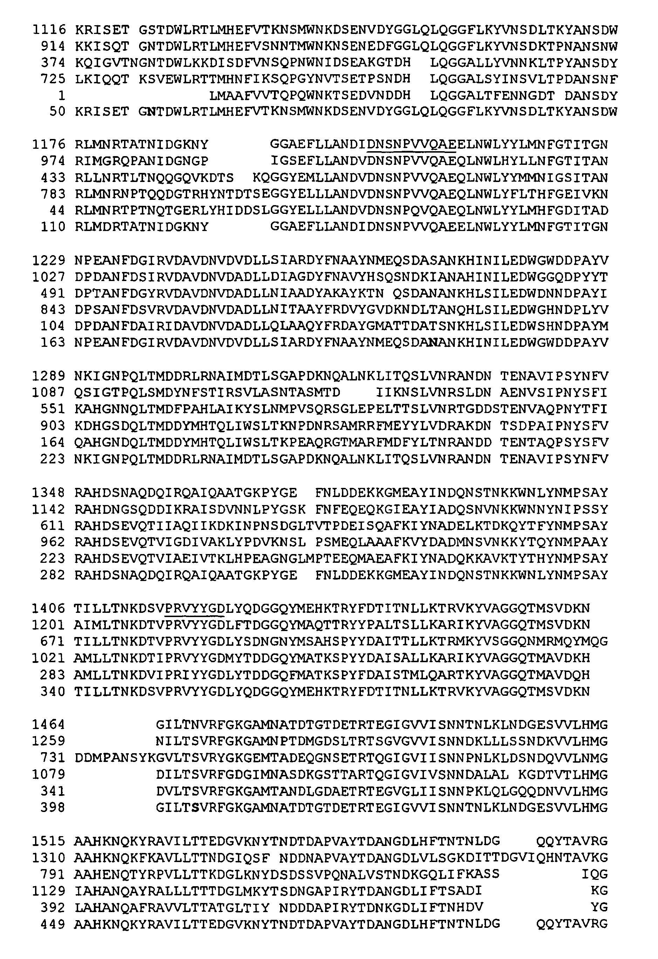

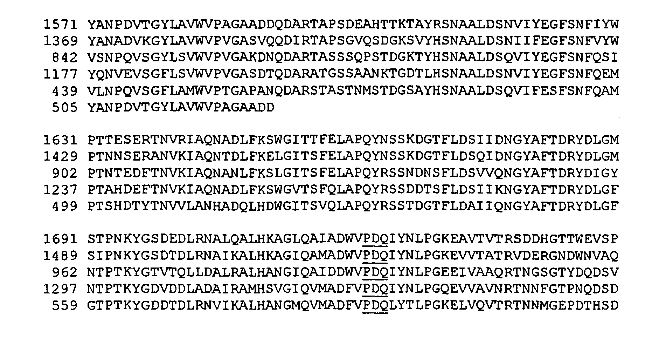

## Claims

1. Use of a glucan comprising 15-26% of α(1,3)-linked AGU, 30-50% of α(1,6)-linked AGU, and 5-20% of α-(1,3,6)-linked AGU, as an anti-corrosion agent.

2. Use according to claim 1, wherein the glucan has an average molecular weight between 10kDa and 50 MDa.

3. Use according to claim 1 or 2, wherein the glucan is capable of being produced by glucosyltransferase activity of *Lactobacillus reuteri* strain 180 deposited as LMG P-18389.

## Patentansprüche

1. Verwendung eines Glucans welches 15-26% α(1,3)-verknüpfter AGU, 30-50% α(1,6)-verknüpfter AGU, und 5-20% α(1,3,6)-verknüpfter AGU umfaßt, als Antikorrosionsmittel.

2. Verwendung gemäß Anspruch 1, wobei das Glucan ein mittleres Molekulargewicht zwischen 10 kDa und 50 MDa besitzt.

3. Verwendung gemäß Anspruch 1 oder 2, wobei das Glucan durch Glucosyltransferase-Aktivität des als LMG P-18389 hintergelegten Stammes *Lactobacillus reuteri* 180 hergestellt werden kann.

## Revendications

1. Utilisation d'un glucane comprenant 15-26% d'unités anhydroglucose (AGU) liées α(1,3), 30-50% d'AGU liées α(1,6), et 5-20% AGU liées α(1,3,6), comme agent anticorrosif.

2. Utilisation selon la revendication 1, dans laquelle le glucane a un poids moléculaire moyen compris entre 10 kDa et 50 MDa.

3. Utilisation selon la revendication 1 ou 2, dans laquelle le glucane peut être produit par l'activité de la glucosyltransférase de la souche *Lactobacillus reuteri* 180 déposée comme LMG P-18389.
